# EUROPÄISCHE PATENTSCHRIFT

(11) **EP 2 117 651 B1**
(45) Veröffentlichungstag und Bekanntmachung des Hinweises auf die Patenterteilung: **20.09.2017**
(21) Anmeldenummer: 08707643.6
(22) Anmeldetag: 09.02.2008
(51) Int. Cl.: A61Q 5/06, A61K 8/04, A61K 8/90, A61K 8/81

(54) **HAARSTYLINGGEL MIT HOHEM LANGZEITHALT**
HAIR STYLING GEL HAVING A LONG-LASTING EFFECT
GEL DE COIFFURE À TENUE LONGUE DURÉE ÉLEVÉE

(30) Priorität: 13.02.2007 DE 102007008089
(43) Veröffentlichungstag der Anmeldung: 18.11.2009
(73) Patentinhaber: Beiersdorf AG, 20253 Hamburg (DE)
(72) Erfinder: HEUER, Björn, 22145 Hamburg (DE); GERJETS, Julia, 22303 Hamburg (DE); ARGEMBEAUX, Horst, 21465 Wentorf (DE); PILZNER, Anke, 22459 Hamburg (DE)
(86) Internationale Anmeldenummer: PCT/EP2008/001004
(87) Internationale Veröffentlichungsnummer: WO 2008/098717

(56) Entgegenhaltungen:
- DE-C2- 10 101 382
- JENNIFER REICHL COLLIN WEE TIN KWEK ALBERT LEE AND PAUL REEVE ROHM AND HAAS COMPANY SPRING HOUSE PENNSYLVANIA U S A ET AL: "AculynTM 88 rheology modifier" RESEARCH DISCLOSURE, MASON PUBLICATIONS, HAMPSHIRE, GB, Bd. 494, Nr. 11, 1. Juni 2005 (2005-06-01), XP007135155 ISSN: 0374-4353
- ROHM AND HAAS: "Acudyne 180 Hair Fixative and Styling Polymer" PERSONAL CARE, STEP COMMUNICATIONS, TUNBRIDGE WELLS, GB, 1. Mai 2003 (2003-05-01), Seiten 1-16, XP003023173 ISSN: 1470-8213
- ANDREA KEENAN OF THE ROHM AND HAAS COMPANY: "Hair styling formulations containing Acudyne 180 hair fixative polymer and Aculyn rheology modifiers" RESEARCH DISCLOSURE, MASON PUBLICATIONS, HAMPSHIRE, GB, Bd. 478, Nr. 8, 1. Februar 2004 (2004-02-01), XP007133362 ISSN: 0374-4353
- MIAO WANG OF THE ROHM AND HAAS COMPANY ET AL: "Mousse formulations containing Acudyne(TM) DHR or Acudyne(TM) 180 hair fixative polymer and Aculyn(TM) 88 rheology modifier" RESEARCH DISCLOSURE, MASON PUBLICATIONS, HAMPSHIRE, GB, Bd. 510, Nr. 27, 1. Oktober 2006 (2006-10-01), Seite 1297, XP007136706 ISSN: 0374-4353
- ROHM AND HAAS: "Personal Care, Suspending Spray Hair Gel" INTERNET CITATION, [Online] XP002414451 Gefunden im Internet: URL:http://www.rohmhaas.com/personalcare/p df/Formulations/0035.pdf> [gefunden am 2007-01-12]

## Beschreibung

Haargele sind eine Klasse der Haarstyling-Produkte, die zur temporären Verformung und Stabilisierung der Haare eingesetzt werden.

Das klassische Haargel ist transparent und weist ein spezielles rheologisches Profil auf, welches sich durch eine hohe Ruheviskosität, eine Fließgrenze und Scherverdünnung (Strukturviskosität) auszeichnet. Dieses rheologische Profil sorgt dafür, dass das Produkt nicht von alleine aus der Tube fließt (Fließgrenze), nach der Entnahme ein stabiles Gel ist (hohe Ruheviskosität) und sich angenehm verteilen lässt (Scherverdünnung).

Dieses rheologische Profil wird im allgemeinen mit Verdickern auf Basis vernetzter Polyacrylsäure (Carbomere, z. B. Carbopol® 980 von Noveon) erreicht, alternative Verdicker sind z. B. Cellulose-, Stärke- oder Guarderivate und hydrophob modifizierte Polyacrylate, die aber zu anderen rheologischen und/oder sensorischen Eigenschaften führen.

Ein wesentlicher Nachteil der vernetzten Polyacrylsäuren ist die Inkompatibilität mit Elektrolyten, die zu einem Zusammenbruch der Netzwerkstruktur des Verdickers führen.

Aus diesem Grund bilden Carbomere mit ionischen Filmbildnern keine klaren, hochviskosen Gele aus, weswegen auf nichtionische Filmbildner, in der Regel basierend auf Vinylpyrrolidon und Vinylacetat zurück gegriffen werden muss.

Diese Systeme sind aus zweierlei Gründen nicht optimal: Zum einen sind die ausgebildeten Filme auf dem Haar sehr spröde, weswegen die Festigung der Frisur bei leichter mechanischer Belastung bereits deutlich verloren geht, zum anderen sind VP/VA Copolymere hygroskopisch, d. h. die Filme werden bei erhöhter Luftfeuchtigkeit schnell weich und somit verliert die Frisur an Festigung.

Ionische, insbesondere anionische, Filmbildner weisen diese Nachteile nicht auf, weswegen Ziel einer Haargelentwicklung ist, ein System zu finden, welches die gewünschten rheologischen und sensorischen Eigenschaften aufweist, dabei aber mit einem anionischen Filmbildner formuliert ist, also auch einen alternativen Verdicker enthalten muss.

Cellulose-, Stärke- oder Guarderivate weisen in typischen Gelformulierungen negative sensorische Eigenschaften auf, weswegen hydrophob modifizierte Acrylatcopolymere (HASE-Verdicker; Hydrophobically modfied Alkali Swellable Emulsions) bevorzugt eingesetzt werden.

Auf dem Markt befindliche Verdicker dieser Art sind z.B. Aculyn® 22 (Acrylates/Steareth-20 Methacrylate Copolymer), Aculyn® 28 (Acrylates/Beheneth-25 Methacrylate Copolymer), Structure 2001® (Acrylates/Steareth-20 Itaconate Copolymer), Structure 3001® (Acrylates/Ceteth-20 Itaconate Copolymer), Structure Plus® (Acrylates/Aminoacrylates C10-30 Alkyl PEG-20 Itaconate Copolymer), Carbopol® 1342, 1382, Ultrez 20, Ultrez 21 (Acrylates/C10-30 Alkyl Acrylate Crosspolymer) oder Synthalen W 2000® (Acrylates/Palmeth-25 Acrylate Copolymer).

Diese Verdicker führen in der Regel durch die nur sehr schwach ausgeprägte Fließgrenze zu Gelen mit ungünstigen rheologischen Eigenschaften, da sie ohne Druck aus der Tube fließen.

Zudem führen die oberflächenaktiven hydrophoben Seitenketten zu einem Aufschäumen des Gels, wenn es auf der Hand und/oder dem Haar verteilt wird.

Um das Aufschäumen zu verhindern, müssen Entschäumer eingesetzt werden, wie z.B. Silikon Copolyole oder Polyalkylene und deren Derivate. Um effektiv gegen das Aufschäumen wirken zu können, müssen diese Entschäumer in so hohen Mengen zugegeben werden, dass sie sich das negativ auf die Filmeigenschaften, insbesondere auf die Festigungsleistung, auswirken.

Die Schrift EP 0 985 405 A2 offenbart die Verwendung von bestimmten Acrylatpolymeren in wässrigen Stylingprodukten. Über Polyalkylenoxy-Copolymere offenbart diese Schrift nichts.

Das Journal of Research Disclosure, Februar 2004, RD Database Number 478008 offenbart die Verwendung von bestimmten Acrylatpolymeren in Kombination mit weiteren Acrylatpolymeren. Über Polyalkylenoxy-Copolymere offenbart diese Schrift nichts.

Das Datenblatt Acudyne® 180, Rohm & Haas (Mai 2000) offenbart die Verwendung von bestimmten Acrylatpolymeren in Kombination mit weiteren Acrylatpolymeren. Über Polyalkylenoxy-Copolymere offenbart diese Schrift nichts.

Das Journal of Research Disclosure, Juni 2005, "Aculyn TM 88 rheology modifier" offenbart die Verwendung von bestimmten Acrylatpolymeren in Kombination mit Sorbitol. Über Polyalkylenoxy-Copolymere offenbart diese Schrift nichts.

Die Schrift EP 1 238 646 B1 (Goldwell) offenbart die Verwendung von bestimmten Acrylatpolymeren in Kombination mit Allylether-Acrylatcopolymeren. Über Polyalkolenoxy-Copolymere offenbart diese Schrift nichts.

Die Schrift US 2003/0165454 A1 (P & G) offenbart Zubereitungen mit vernetzten Polyacrylatverdickern und "Frizz-Control"-Mittel wie Silikon Copolyol. Über Polyalkolenoxy-Copolymere offenbart diese Schrift nichts.

Die Schrift WO 1997/046213 (Unilever) offenbart die Verwendung von Silikon Copolyol als Entschäumer in Haarsprays, jedoch nichts über wässrige Gele.

Die Schrift EP 0 953 015 B1 (L'Oréal) offenbart die Verwendung von speziellen Verdicker mit Silikon.

### Erfindung

Es hat sich nun überraschender Weise gezeigt, dass die im Stand der Technik beschriebenen Probleme gelöst werden können durch eine kosmetische Zubereitung enthaltend a) mindestens ein anionisches, filmbildendes Polymer, wobei das anionische, filmbildende Polymer ausgesucht ist unter linearen, statistischen Acrylat-Copolymeren, enthaltend (Meth-)Acrylsäure, Alkyl (Meth-)Acrylate und weitere Monomere, b) mindestens einen hydrophob modifizierten Acrylat-Verdicker, wobei der hydrophob modifizierte der Acrylat-Verdicker zusätzlich quervernetzt ist und c) mindestens ein Blockpolymer aufgebaut aus mindestens einem Polyethylenglykol- und mindestens einem Polypropylenglykol-Block, sowie d) gegebenenfalls ein Silikon Copolyol. Besonders geeignget ist die Kombination Aculyn® 88 (hydrophob modifizierter, quervernetzter Polyacrylat-Verdicker) mit Acudyne® 180, einem linearen, anionischen Acrylat-Copolymer, das aus mindestens drei Monomeren statistisch aufgebaut ist und geringen Mengen Pluronic® L81. Besonders vorteilhaft ist es, wenn der Formulierung noch eine geringe Menge eines Silikon Copolyols zugegeben wird.

Das Resultat ist ein Haarstylinggel, welches überlegenen Langzeithalt aufweist, ein gutes rheologisches und sensorisches Verhalten zeigt und beim Verteilen in der Hand und auf dem Haar praktisch nicht schäumt. Daher wird das anionische, filmbildende Polymer ausgesucht unter den linearen, statistischen Acrylat-Copolymeren, enthaltend (Meth-) Acrylsäure, Alkyl (Meth-)Acrylate und gegebenenfalls weitere Monomere. Weiter ist es bevorzugt, wenn das filmbildende Polymer in einer Konzentration zwischen 0,5 und 5 % eingesetzt wird. Der hydrophob modifizierte Acrylat-Verdicker ist zusätzlich quervernetzt. Weiter ist es bevorzugt, wenn der hydrophob modifizierte Acrylat-Verdicker in einer Konzentration zwischen 0,5 und 3 % eingesetzt wird. Weiter ist es bevorzugt, wenn das Blockpolymer ein Triblock-Copolymer ist. Dabei ist besonders bevorzugt, wenn das Triblock-Copolymer ein Polypropylenglykol-Polyethylenglykol-Polypropylenglykol Copolymer ist. Ganz besonders bevorzugt ist es, wenn das Triblock-Copolymer in einer Konzentration zwischen 0,01 und 0,5% eingesetzt wird. Die Erfindung umfasst daher die Verwendung von oben beschriebenen Zubereitungen zur Haarbehandlung und insbesondere zur Gestaltung von Frisuren.

### Vorteilhafte Ausführungsformen

Es ist erfindungsgemäß vorteilhaft, wenn die erfindungsgemäße Zubereitung in Form eines klaren Gels vorliegt, welches gekennzeichnet ist durch eine Viskosität zwischen 5000 und 50000 mPa·s.

Es ist ferner vorteilhaft, wenn das Gel frei von Hydroxyalkanen, insbesondere Ethanol ist. Die erfindungsgemäßen Zubereitungen können kosmetische Hilfsstoffe enthalten wie Konservierungsmittel, Antioxidantien, Bakterizide, Parfüme, Emulgatoren, Tenside, Farbstoffe, Pigmente, Partikel, Gasbläschen, weitere Rheologie oder Sensorik modifizierende Polymere, quaternäre Verbindungen und Polymere, Alkohole, mehrwertige Alkohole, Polyole, Stabilisatoren, Silikone, Silikon-Copolyole oder andere Silikonderivate, Komplexbildner, UV-Filter, organische Lösungsmittel und Treibmittel. Die aufgeführte Liste an Zusatzstoffen ist im Rahmen der Erfindung nicht limitierend.

### Beispiele

Zur Untersuchug der offenbarten Rezepturen wurden folgende Methoden verwendet:

### Fließgrenze:

Platte/Platte-Rheometer 25mm, 1 mm Spalt, 25°C, 0 bis 400 Pa bei 40 Pa/min

### Klarheit:

Visuelle Beurteilung

### Halt:

Zyklischer 3-Punkt Biegeversuch. Die Haarsträhne (20 cm, 2g Gewicht) wird mit 1g Produkt behandelt und bei 25°C und 55% r.F. für mindestens 12 Stunden konditioniert. Anschließend wird die Strähne in einem 3-Punkt Biegeversuch wiederholt um eine gleichmäßig zunehmende Strecke gedehnt und die dafür benötigte Kraft gemessen. Aus der so maximal benötigten Kraft wird die Halteleistung ermittelt und in einer 4er-Skala bewertet:
1: Fₘₐₓ < 500 mN
2: Fₘₐₓ 500...750 mN
3: Fₘₐₓ 750...1000 mN
4: Fₘₐₓ > 1000 mN

Die Elastizität wird über die Auslenkung beschrieben, bei der die maximale Kraft auftritt:
1: Smax < 1 mm
2: Sₘₐₓ 1...2 mm
3: Smax 2...3 mm
4: Smax 3...4 mm
5: Smax 4...5 mm
6: Sₘₐₓ > 5 mm

High Humidity Curl Retention (HHCR) bei 25°C und 90% rel. Luftfeuchtigkeit. Die Bewertung erfolgt in einer 3er-Skala:
1: HHCR < 25%
2: HHCR = 25...50%
3: HHCR > 50%

### Schäumen:

Experten-Panel, Bewertung der Schaumbildung beim Verreiben des Gels zwischen den Händen. Dazu werden 3,5g Gel in die eine Handfläche gegeben und zwischen beiden Händen 5 Sekunden lang intensiv verrieben. Anschließend wird beurteilt, ob sich Schaum gebildet hat. Dann wird das Produkt zwischen den Händen wiederum 5 Sekunden lang intensiv verrieben und die Bewertung erneut durchgeführt. Dieser Vorgang wird insgesamt fünfmal wiederholt und das Ergebnis von den Panelisten entsprechend der Vorgaben bewertet:
1: Auch nach dem fünften Verreiben ist kein Schaum zu erkennen
2: Leichte Schaumbildung nach dem dritten, vierten oder fünften Verreiben
3: Schaumbildung nach dem zweiten Verreiben
4: Deutliche Schaumbildung bereits nach dem ersten Verreiben

**Zubereitungen:**

| | 1 | 2* | 3 | 4* | 5 | 6 | 7 | 8* | 9 | 10 | 11 |
|---|---|---|---|---|---|---|---|---|---|---|---|
| VP/VA Copolymer ¹⁾ | 4 | | | | | | | | | | |
| Acrylates/Hydroxyesters Acrylates Copolymer ²⁾ | | 1,5 | 1,5 | 1,5 | 1,5 | 1,5 | 1,5 | | | 1,5 | 1,5 |
| Acrylates Copolymer ³⁾ | | | | | | | | 1,5 | | | |
| Octylacrylamide/Acrylates/ Butylaminoethyl Methacrylates Copolymer ⁴⁾ | | | | | | | | | 1,5 | | |
| Carbomer ⁵⁾ | 0.7 | | | | | | | | | | |
| Acrylates/Steareth-20 Methacrylate Crosspolymer ⁶⁾ | | 1,5 | 1,5 | 1,5 | 1,5 | 1,5 | 1,5 | 1,5 | 1,5 | | |
| Acrylates/Beheneth-25 Methacrylate Copolymer ⁷⁾ | | | | | | | | | | 1,5 | |
| Acrylates/Steareth-20 Metacrylate Copoylmer ⁸⁾ | | | | | | | | | | | 1,5 |
| Poloxamer 231 ⁹⁾ | | 0,1 5 | | 0,5 | | | | 0,1 5 | 0,1 5 | 0,1 5 | 0,1 5 |
| PEG/PPG-20/20 Dimethicone ¹⁰⁾ | | 0,1 5 | 0,5 | | | | | 0,1 5 | 0,1 5 | 0,1 5 | 0,1 5 |
| PEG/PPG-20/6 Dimethicone ¹¹⁾ | | | | | 0,5 | | | | | | |
| PEG/PPG-17/18 Dimethicone ¹²⁾ | | | | | | 0,5 | | | | | |
| PEG-12 Dimethicone ¹³⁾ | | | | | | | 0,5 | | | | |
| DMDM Hydantoin | 0,2 | 0,2 | 0,2 | 0,2 | 0,2 | 0,2 | 0,2 | 0,2 | 0,2 | 0,2 | 0,2 |
| Fragrance | 0,2 | 0,2 | 0,2 | 0,2 | 0,2 | 0,2 | 0,2 | 0,2 | 0,2 | 0,2 | 0,2 |
| PEG-40 Hydrogenated Castor Oil | 0,4 | 0,4 | 0,4 | 0,4 | 0,4 | 0,4 | 0,4 | 0,4 | 0,4 | 0,4 | 0,4 |
| Water | Ad 10 0 | Ad 10 0 | Ad 10 0 | Ad 10 0 | Ad 10 0 | Ad 10 0 | Ad 10 0 | Ad 10 0 | Ad 10 0 | Ad 10 0 | Ad 10 0 |
| | | | | | | | | | | | |
| Klarheit | klar | klar | klar | trü b | klar | klar | klar | klar | klar | klar | klar |
| Schäumen | 1 | 2 | 3 | 2 | 4 | 4 | 4 | 2 | 2 | 2 | 2 |
| Halt (max.Biegespannung Fmax) | 2 | 4 | 3 | 4 | 3 | 3 | 3 | 4 | 4 | 4 | 4 |
| Halt (Elastizität) | 2 | 4 | 4 | 4 | 4 | 4 | 4 | 3 | 4 | 4 | 4 |
| Halt (HHCR) | 1 | 3 | 3 | 3 | 3 | 3 | 3 | 3 | 3 | 3 | 3 |

| | | | | | | | | | | | |
|---|---|---|---|---|---|---|---|---|---|---|---|
| *erfindungsgemäß ¹⁾ Luviskol® VA 64 W (BASF) ²⁾ Acudyne® 180 (Rohm & Haas) ³⁾ Luvimer® Pro 55 (BASF) ⁴⁾ Amphomer® LV-71 (National Starch) ⁵⁾ Carbopol® 980 (Noveon) ⁶⁾ Aculyn® 88 (Rohm & Haas) ⁷⁾ Aculyn® 28 (Rohm & Haas) ⁸⁾ Aculyn® 22 (Rohm & Haas) ⁹⁾ Pluronic® L-81 (BASF) ¹⁰⁾ Abil B 8863 (Goldschmidt) ¹¹⁾ Abil B 88183 (Goldschmidt) ¹²⁾ Q2-5220 Resin Modifier (Dow Corning) ¹³⁾ DC 193 Fluid (Dow Corning) | | | | | | | | | | | |

## Patentansprüche

1. Haarstylinggel enthaltend
a) mindestens ein anionisches, filmbildendes Polymer, wobei das anionische, filmbildende Polymer ausgesucht ist unter linearen, statistischen Acrylat-Copolymeren, enthaltend (Meth-)Acrylsäure, Alkyl (Meth-)Acrylate und weitere Monomere,
b) mindestens einen hydrophob modifizierten Acrylat-Verdicker, wobei der hydrophob modifizierte der Acrylat-Verdicker zusätzlich quervernetzt ist, und
c) mindestens ein Blockpolymer, aufgebaut aus mindestens einem Polyethylenglykol- und mindestens einem Potypropylenglykol-Block, sowie
d) gegebenenfalls ein Silikon Copolyol.

2. Haarstylinggel nach Anspruch 1, wobei das filmbildende Polymer in einer Konzentration zwischen 0,5 und 5 % eingesetzt wird.

3. Haarstylinggel nach einem der vorangehenden Patentansprüche, wobei der hydrophob modifizierte Acrylat-Verdicker in einer Konzentration zwischen 0,5 und 3% eingesetzt wird.

4. Haarstylinggel nach einem der vorangehenden Patentansprüche, wobei das Blockpolymer ein Triblock-Copolymer ist.

5. Haarstylinggel nach Patentanspruch 4, wobei das Triblock-Copolymer ein Polypropylenglykol-Polyethylenglykol-Polypropylenglykol Copolymer ist.

6. Haarstylinggel nach Patentanspruch 4 oder 5, wobei das Triblock-Copolymer in einer Konzentration zwischen 0,01 und 0,5 % eingesetzt wird.

7. Verwendung von Zubereitungen nach einem der vorangehenden Patentansprüche zur Haarbehandlung.

8. Verwendung von Zubereitungen nach einem der vorangehenden Patentansprüche zur Gestaltung von Frisuren.

## Claims

1. Hair styling gel comprising
a) at least one anionic film-forming polymer, wherein the anionic film-forming polymer is selected from linear, statistical acrylate copolymers comprising (meth)acrylic acid, alkyl (meth)acrylates and further monomers,
b) at least one hydrophobically modified acrylate thickener, wherein the hydrophobically modified acrylate thickener is additionally cross-linked, and
c) at least one block polymer, composed of at least one polyethylene glycol block and at least one polypropylene glycol block, and
d) optionally a silicone copolyol.

2. Hair styling gel according to Claim 1, wherein the film-forming polymer is used at a concentration between 0.5 and 5%.

3. Hair styling gel according to either of the preceding patent claims, wherein the hydrophobically modified acrylate thickener is used at a concentration between 0.5 and 3%.

4. Hair styling gel according to any of the preceding patent claims, wherein the block polymer is a triblock copolymer.

5. Hair styling gel according to Patent Claim 4, wherein the triblock copolymer is a polypropylene glycol-polyethylene glycol-polypropylene glycol copolymer.

6. Hair styling gel according to Patent Claim 4 or 5, wherein the triblock copolymer is used at a concentration between 0.01 and 0.5%.

7. Use of preparations according to any of the preceding patent claims for treating hair.

8. Use of preparations according to any of the preceding patent claims for shaping hairstyles.

## Revendications

1. Gel de coiffure contenant :
a) au moins un polymère filmogène anionique, le polymère filmogène anionique étant choisi parmi les copolymères d'acrylate statistiques linéaires contenant l'acide (méth)acrylique, les (méth)acrylates d'alkyle et d'autres monomères,
b) au moins un épaississant acrylate modifié hydrophobiquement, l'épaississant acrylate modifié hydrophobiquement étant en outre réticulé, et
c) au moins un polymère séquencé, formé par au moins une séquence polyéthylène glycol et au moins une séquence polypropylène glycol, ainsi que
d) éventuellement un silicone-copolyol.

2. Gel de coiffure selon la revendication 1, dans lequel le polymère filmogène est utilisé en une concentration comprise entre 0,5 et 5 %.

3. Gel de coiffure selon l'une quelconque des revendications précédentes, dans lequel l'épaississant acrylate modifié hydrophobiquement est utilisé en une concentration comprise entre 0,5 et 3 %.

4. Gel de coiffure selon l'une quelconque des revendications précédentes, dans lequel le polymère séquencé est un copolymère triséquencé.

5. Gel de coiffure selon la revendication 4, dans lequel le copolymère triséquencé est un copolymère de polypropylène glycol-polyéthylène glycol-polypropylène glycol.

6. Gel de coiffure selon la revendication 4 ou 5, dans lequel le copolymère triséquencé est utilisé en une concentration comprise entre 0,01 et 0,5 %.

7. Utilisation de préparations selon l'une quelconque des revendications précédentes pour le traitement de cheveux.

8. Utilisation de préparations selon l'une quelconque des revendications précédentes pour la formation de coiffures.
